# EUROPEAN PATENT SPECIFICATION

(11) **EP 3 743 053 B1**
(45) Date of publication and mention of the grant of the patent: **29.11.2023**
(21) Application number: 19702670.1
(22) Date of filing: 22.01.2019
(51) Int. Cl.: A61K 31/05, A61K 31/36, A61K 31/551, A61K 45/06, A61P 25/08

(54) **USE OF CANNABINOIDS IN THE TREATMENT OF EPILEPSY**
VERWENDUNG VON CANNABINOIDEN BEI DER BEHANDLUNG VON EPILEPSIE
UTILISATION DE CANNABINOÏDES DANS LE TRAITEMENT DE L'ÉPILEPSIE

(30) Priority: 24.01.2018 GB 201801158
(43) Date of publication of application: 02.12.2020
(73) Proprietor: GW Research Limited, Cambridge, Cambridgeshire CB24 9BZ (GB)
(72) Inventor: GUY, Geoffrey, Cambridgeshire CB24 9BZ (GB); KNAPPERTZ, Volker, Cambridgeshire CB24 9BZ (GB); WHALLEY, Benjamin, Cambridgeshire CB24 9BZ (GB)
(74) Representative: HGF
(86) International application number: PCT/GB2019/050174
(87) International publication number: WO 2019/145700

(56) References cited:
- WO-A1-2016/059403
- GB-A- 2 539 472
- US-A1- 2017 181 982
- S. WRIGHT ET AL.: "CANNABIDIOL (CBD) IN DRAVET SYNDROME: A RANDOMISED, DOSE-RANGING PHARMACOKINETICS AND SAFETY TRIAL (GWPCARE1)", EPILEPSIA, vol. 58(Suppl.5), September 2017 (2017-09) , page s56, XP002790754,
- EMILIO PERUCCA: "Cannabinoids in the Treatment of Epilepsy: Hard Evidence at Last?", JOURNAL OF EPILEPSY RESEARCH, vol. 7, no. 2, 28 December 2017 (2017-12-28), pages 61-76, XP55581907, ISSN: 2233-6249, DOI: 10.14581/jer.17012
- ORRIN DEVINSKY ET AL: "Trial of Cannabidiol for Drug-Resistant Seizures in the Dravet Syndrome", THE NEW ENGLAND JOURNAL OF MEDICINE, - NEJM -, vol. 376, no. 21, 25 May 2017 (2017-05-25) , pages 2011-2020, XP55581640, US ISSN: 0028-4793, DOI: 10.1056/NEJMoa1611618
- GEFFREY A L ET AL: "Drug-drug interaction between clobazam and cannabidiol in children with refractory epilepsy", EPILEPSIA 20150801 BLACKWELL PUBLISHING INC. USA, vol. 56, no. 8, 1 August 2015 (2015-08-01) , pages 1246-1251, XP002790755, ISSN: 0013-9580
- EVAN J. HESS ET AL: "Cannabidiol as a new treatment for drug-resistant epilepsy in tuberous sclerosis complex", EPILEPSIA, vol. 57, no. 10, 1 October 2016 (2016-10-01), pages 1617-1624, XP55501922, NEW YORK, US ISSN: 0013-9580, DOI: 10.1111/epi.13499

## Description

### FIELD OF THE INVENTION

The present invention relates to cannabidiol (CBD) for use in the treatment of patients with childhood-onset epilepsy who are concurrently taking stiripentol.

Preferably the CBD used is in the form of a highly purified extract of cannabis such that the CBD is present at greater than 98% of the total extract (w/w) and the other components of the extract are characterised. In particular the cannabinoid tetrahydrocannabinol (THC) has been substantially removed, to a level of not more than 0.15% (w/w) and the propyl analogue of CBD, cannabidivarin, (CBDV) is present in amounts of up to 1%. Alternatively, the CBD may be a synthetically produced CBD.

### BACKGROUND TO THE INVENTION

Epilepsy occurs in approximately 1% of the population worldwide, (Thurman *et al.,* 2011) of which 70% are able to adequately control their symptoms with the available existing anti-epileptic drugs (AEDs). However, 30% of this patient group, (Eadie *et al.,* 2012), are unable to obtain seizure freedom from the AED that are available and as such are termed as suffering from intractable or "treatment-resistant epilepsy" (TRE).

Intractable or treatment-resistant epilepsy was defined in 2009 by the International League Against Epilepsy (ILAE) as "failure of adequate trials of two tolerated and appropriately chosen and used AED schedules (whether as monotherapies or in combination) to achieve sustained seizure freedom" (Kwan et al., 2009).

Individuals who develop epilepsy during the first few years of life are often difficult to treat and as such are often termed treatment-resistant. Children who undergo frequent seizures in childhood are often left with neurological damage which can cause cognitive, behavioral and motor delays.

Childhood-onset epilepsy is a relatively common neurological disorder in children and young adults with a prevalence of approximately 700 per 100,000. This is twice the number of epileptic adults per population.

When a child or young adult presents with a seizure, investigations are normally undertaken in order to investigate the cause. Childhood epilepsy can be caused by many different syndromes and genetic mutations and as such diagnosis for these children may take some time.

The main symptom of epilepsy is repeated seizures. In order to determine the type of epilepsy or the epileptic syndrome that a patient is suffering from, an investigation into the type of seizures that the patient is experiencing is undertaken. Clinical observations and electroencephalography (EEG) tests are conducted and the type(s) of seizures are classified according to the ILAE classification described below.

The International classification of seizure types proposed by the ILAE was adopted in 1981 and a revised proposal was published by the ILAE in 2010 and has not yet superseded the 1981 classification. The 2010 proposal for revised terminology includes the proposed changes to replace the terminology of partial with focal. In addition, the term "simple partial seizure" has been replaced by the term "focal seizure where awareness / responsiveness is not impaired" and the term "complex partial seizure" has been replaced by the term "focal seizure where awareness / consciousness is impaired".

Generalised seizures, where the seizure arises within and rapidly engages bilaterally distributed networks, can be split into six subtypes: Tonic-Clonic (grand mal) seizures; Absence (petit mal) Seizures; Clonic Seizures; Tonic Seizures; Atonic Seizures and Myoclonic Seizures.

Focal (partial) seizures where the seizure originates within networks limited to only one hemisphere, are also split into sub-categories. Here the seizure is characterized according to one or more features of the seizure, including aura, motor, autonomic and awareness / responsiveness. Where a seizure begins as a localized seizure and rapidly evolves to be distributed within bilateral networks this seizure is known as a Bilateral convulsive seizure, which is the proposed terminology to replace Secondary Generalised Seizures (generalized seizures that have evolved from focal seizures and are no longer remain localized).

Epileptic syndromes often present with many different types of seizure and identifying the types of seizure that a patient is suffering from is important as many of the standard AEDs are targeted to treat or are only effective against a given seizure type / subtype.

One such childhood epilepsy syndrome is Lennox-Gastaut syndrome (LGS). LGS is a severe form of epilepsy, where seizures usually begin before the age of 4. Seizure types, which vary among patients, include tonic (stiffening of the body, upward deviation of the eyes, dilation of the pupils, and altered respiratory patterns), atonic (brief loss of muscle tone and consciousness, causing abrupt falls), atypical absence (staring spells), and myoclonic (sudden muscle jerks). There may be periods of frequent seizures mixed with brief, relatively seizure-free periods.

Seizures in LGS are often described as "drop seizures". Such drop seizures are defined as an attack or spell (atonic, tonic or tonic-clonic) involving the entire body, trunk or head that led or could have led to a fall, injury, slumping in a chair or hitting the patient's head on a surface.

Most patients with LGS experience some degree of impaired intellectual functioning or information processing, along with developmental delays, and behavioural disturbances.

LGS can be caused by brain malformations, perinatal asphyxia, severe head injury, central nervous system infection and inherited degenerative or metabolic conditions. In 30-35% of cases, no cause can be found.

The first line treatment for drop seizures, including the treatment of drop seizures in patients with LGS, usually comprises a broad-spectrum AED, such as sodium valproate often in combination with rufinamide or lamotrigine. Other AEDs that may be considered include felbamate, clobazam and topiramate.

Another childhood epilepsy syndrome is Dravet syndrome. Onset of Dravet syndrome almost always occurs during the first year of life with clonic and tonic-clonic seizures in previously healthy and developmentally normal infants (Dravet, 2011). Symptoms peak at about five months of age. Other seizures develop between one and four years of age such as prolonged focal dyscognitive seizures and brief absence seizures.

In diagnosing Dravet syndrome both focal and generalised seizures are considered to be mandatory, Dravet patients may also experience atypical absence seizures, myoclonic absence seizures, atonic seizures and non-convulsive status epilepticus.

Seizures progress to be frequent and treatment-resistant, meaning that the seizures do not respond well to treatment. They also tend to be prolonged, lasting more than 5 minutes. Prolonged seizures may lead to status epilepticus, which is a seizure that lasts more than 30 minutes, or seizures that occur in clusters, one after another.

Prognosis is poor and approximately 14% of children die during a seizure, because of infection, or suddenly due to uncertain causes, often because of the relentless neurological decline. Patients develop intellectual disability and life-long ongoing seizures. Intellectual impairment varies from severe in 50% patients, to moderate and mild intellectual disability each accounting for 25% of cases.

There are currently no FDA approved treatments specifically indicated for Dravet syndrome. The standard of care usually involves a combination of the following anticonvulsants: clobazam, clonazepam, levetiracetam, topiramate and valproic acid.

Stiripentol is approved in Europe for the treatment of Dravet syndrome in conjunction with clobazam and valproic acid. In the US, stiripentol was granted an Orphan Designation for the treatment of Dravet syndrome in 2008; however, the drug is not FDA approved.

Potent sodium channel blockers used to treat epilepsy actually increase seizure frequency in patients with Dravet Syndrome. The most common are phenytoin, carbamazepine, lamotrigine and rufinamide.

Management may also include a ketogenic diet, and physical and vagus nerve stimulation. In addition to anti-convulsive drugs, many patients with Dravet syndrome are treated with anti-psychotic drugs, stimulants, and drugs to treat insomnia.

Orrin Devinsky et al.: The New England Journal of Medicine; Vol.376(11); pages 2011-2020, discloses a trial of cannabidiol for drug-resistant seizures in the Dravet syndrome. Cannabidiol resulted in a greater reduction in convulsive-seizure frequency.

Common AEDs defined by their mechanisms of action are described in the following tables:

**Table 1. Examples of narrow spectrum AEDs**

| **Narrow-spectrum AED** | **Mechanism** | **Indication** |
|---|---|---|
| Phenytoin | Sodium channel | Complex partial |
| | | Tonic-clonic |
| Phenobarbital | GABA / Calcium channel | Partial seizures |
| | | Tonic-clonic |
| Carbamazepine | Sodium channel | Partial seizures |
| | | Tonic-clonic |
| | | Mixed seizures |
| Oxcarbazepine | Sodium channel | Partial seizures |
| | | Tonic-clonic |
| | | Mixed seizures |
| Gabapentin | Calcium channel | Partial seizures |
| | | Mixed seizures |
| Pregabalin | Calcium channel | Adjunct therapy for partial seizures with or without secondary generalisation |
| Lacosamide | Sodium channel | Adjunct therapy for partial seizures |
| Vigabatrin | GABA receptor agonism | Secondarily generalized tonic-clonic seizures |
| | | Partial seizures |
| | | Infantile spasms due to West syndrome |

**Table 2. Examples of broad spectrum AEDs**

| **Broad-spectrum AED** | **Mechanism** | **Indication** |
|---|---|---|
| Valproic acid | GABA / Sodium channel | First-line treatment for tonic-clonic seizures, absence seizures and myoclonic seizures |
| | | Second-line treatment for partial seizures and infantile spasms. |
| | | Intravenous use in status epilepticus |
| Lamotrigine | Sodium channel | Partial seizures |
| | | Tonic-clonic |
| | | Seizures associated with Lennox-Gastaut syndrome |
| Ethosuximide | Calcium channel | Absence seizures |
| Topiramate | GABA / Sodium channel | Seizures associated with Lennox-Gastaut syndrome |
| Zonisamide | GABA / Calcium /Sodium channel | Adjunctive therapy in adults with partial-onset seizures Infantile spasm |
| | | Mixed seizure Lennox-Gastaut syndrome Myoclonic |
| | | Generalised tonic-clonic seizure |
| Levetiracetam | Calcium channel | Partial seizures |
| | | Adjunctive therapy for partial, myoclonic and tonic-clonic seizures |
| Clonazepam | GABA receptor agonism | Typical and atypical absences |
| | | Infantile myoclonic |
| | | Myoclonic seizures |
| | | Akinetic seizures |
| Rufinamide | Sodium channel | Adjunctive treatment of partial seizures associated with Lennox-Gastaut syndrome |

**Table 3. Examples of AEDs used specifically in childhood epilepsy**

| **AED** | **Mechanism** | **Indication** |
|---|---|---|
| Clobazam | GABA receptor agonism | Adjunctive therapy in complex partial seizures |
| | | Status epilepticus |
| | | Myoclonic |
| | | Myoclonic-absent |
| | | Simple partial |
| | | Complex partial |
| | | Absence seizures |
| | | Lennox-Gastaut syndrome |
| Stiripentol | GABA receptor agonism | Severe myoclonic epilepsy in infancy (Dravet syndrome) |

The present invention demonstrates that patients with treatment-resistant childhood onset epilepsy have a poor response rate for seizure reduction when treated with the anti-epileptic drug stiripentol. Furthermore, the efficacy of the treatment with stiripentol is found to be reduced further when combined with the anti-epileptic drug clobazam. However, surprisingly when the cannabinoid cannabidiol (CBD) is provided in combination with either stiripentol or stiripentol and clobazam there is a significant and beneficial increase in the response rate in the ability of the drugs to reduce seizures.

Such an increase in efficacy by the addition of CBD is unexpected, particularly given the data that demonstrates a decrease in efficacy when the stiripentol is combined with clobazam.

### BRIEF SUMMARY OF THE DISCLOSURE

In accordance with a first aspect of the present invention there is provided cannabidiol (CBD) for use in the reduction of seizures in treatment-resistant epilepsy, wherein the CBD is administered in combination with stiripentol.

In a further embodiment the CBD is administered in combination with stiripentol and clobazam.

Preferably the CBD is in the form of a highly purified extract of cannabis which comprises at least 98% (w/w) CBD and comprises less than 0.15% THC and up to 1% CBDV.

Alternatively, the CBD is present as a synthetic compound.

Preferably the dose of CBD is below 50 mg/kg/day. More preferably wherein the dose of CBD is greater than 10 mg/kg/day, more preferably the dose of CBD is greater than 20 mg/kg/day.

The treatment-resistant epilepsy is Dravet Syndrome.

In accordance with a non-claimed aspect of the present disclosure there is provided a method of treating treatment-resistant epilepsy, wherein the CBD is administered to an individual in need thereof in combination with stiripentol (STP).

Preferably the individual is a human.

### DEFINITIONS

Definitions of some of the terms used to describe the invention are detailed below:

The cannabinoids described in the present application are listed below along with their standard abbreviations.

**Table 4. Cannabinoids and their abbreviations**

| | | |
|---|---|---|
| CBD | Cannabidiol | 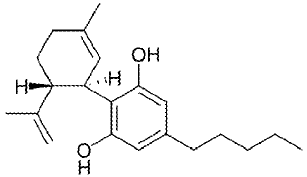 |
| CBDA | Cannabidiolic acid | 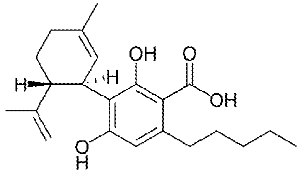 |
| CBDV | Cannabidivarin | 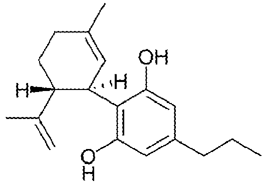 |
| CBDVA | Cannabidivarinic acid | 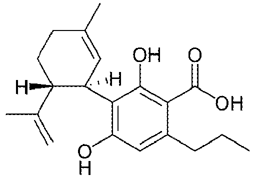 |
| THC | Tetrahydrocannabinol | 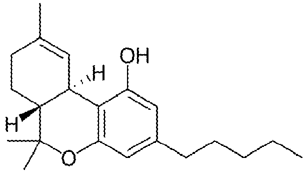 |

The table above is not exhaustive and merely details the cannabinoids which are identified in the present application for reference. So far over 60 different cannabinoids have been identified and these cannabinoids can be split into different groups as follows: Phytocannabinoids; Endocannabinoids and Synthetic cannabinoids (which may be novel cannabinoids or synthetically produced phytocannabinoids or endocannabinoids).

"Phytocannabinoids" are cannabinoids that originate from nature and can be found in the cannabis plant. The phytocannabinoids can be isolated from plants to produce a highly purified extract or can be reproduced synthetically.

"Highly purified cannabinoid extracts" are defined as cannabinoids that have been extracted from the cannabis plant and purified to the extent that other cannabinoids and non-cannabinoid components that are co-extracted with the cannabinoids have been substantially removed, such that the highly purified cannabinoid is greater than or equal to 98% (w/w) pure.

"Synthetic cannabinoids" are compounds that have a cannabinoid or cannabinoid-like structure and are manufactured using chemical means rather than by the plant.

Phytocannabinoids can be obtained as either the neutral (decarboxylated form) or the carboxylic acid form depending on the method used to extract the cannabinoids. For example, it is known that heating the carboxylic acid form will cause most of the carboxylic acid form to decarboxylate into the neutral form.

"Treatment-resistant epilepsy" (TRE) or "intractable epilepsy" is defined as per the ILAE guidance of 2009 as epilepsy that is not adequately controlled by trials of one or more AED. Treatment resistant epilepsies such as Dravet syndrome or Lennox-Gastaut syndrome are difficult to treat childhood epilepsies. Often the treatment of seizures in patients with these syndromes involves the use of adjunctive therapy, e.g., treatment with more than one anti-epileptic drugs concurrently.

"Childhood epilepsy" refers to the many different syndromes and genetic mutations that can occur to cause epilepsy in childhood. Examples of some of these are as follows: Dravet Syndrome; Myoclonic-Absence Epilepsy; Lennox-Gastaut syndrome; Generalized Epilepsy of unknown origin; CDKL5 mutation; Aicardi syndrome; tuberous sclerosis complex; bilateral polymicrogyria; Dup15q; SNAP25; and febrile infection related epilepsy syndrome (FIRES); benign rolandic epilepsy; juvenile myoclonic epilepsy; infantile spasm (West syndrome); and Landau-Kleffner syndrome. The list above is non-exhaustive as many different childhood epilepsies exist.

### DETAILED DESCRIPTION

### PREPARATION OF HIGHLY PURIFIED CBD EXTRACT

The following describes the production of the highly-purified (>98% w/w) cannabidiol extract which has a known and constant composition was used in the Examples below.

In summary the drug substance used is a liquid carbon dioxide extract of high-CBD containing chemotypes of *Cannabis sativa* L. which had been further purified by a solvent crystallization method to yield CBD. The crystallisation process specifically removes other cannabinoids and plant components to yield greater than 98% CBD. Although the CBD is highly purified because it is produced from a cannabis plant rather than synthetically there is a small number of other cannabinoids which are co-produced and co-extracted with the CBD. Details of these cannabinoids and the quantities in which they are present in the medication are as described in Table 5 below.

**Table 5: Composition of highly purified CBD extract**

| **Cannabinoid** | **Concentration** |
|---|---|
| CBD | > 98% w/w |
| CBDA | NMT 0.15% w/w |
| CBDV | NMT 1.0% w/w |
| Δ⁹ THC | NMT 0.15% w/w |
| CBD-C4 | NMT 0.5% w/w |

| | |
|---|---|
| > - greater than NMT - not more than | |

### EXAMPLE 1: DRUG-DRUG INTERACTION BETWEEN CANNABIDIOL (CBD) AND STIRIPENTOL (STP) DURING A CLINICAL TRIAL

The efficacy of CBD for the adjunctive treatment of seizures associated with Dravet syndrome was demonstrated in a single trial in patients aged 2 -18 years. Following completion of a 4-week baseline period, patients were randomized to receive either 20 mg/kg/day CBD (n=61) or placebo (n=59). CBD or placebo were added to their current anti-epileptic treatment which remained stable over the treatment period of the study.

All patients had a diagnosis of treatment-resistant Dravet syndrome and seizures were inadequately controlled with one or more concomitant anti-epileptic drugs (AEDs) with or without vagal nerve stimulation or ketogenic diet.

Seizure counts were reported daily via an Interactive Voice Response System. Convulsive seizures were defined as all countable atonic, tonic, clonic, and tonic-clonic seizures. The primary efficacy end point was percent change from baseline in convulsive seizures.

At baseline, disease state characteristics were comparable between groups with 72.5% reporting an increase in seizure frequency with prior treatment and 15% never experiencing a reduction in seizure frequency with previous medications.

During a 4-week baseline period, patients were required to have at least 4 convulsive seizures (tonic-clonic, clonic, tonic or atonic) while on stable AED therapy. Patients had previously failed a median of 4 prior AEDs and 93% were taking 2 or more concomitant AEDs during the trial. The most commonly used concomitant AEDs (>25% of patients) were clobazam, valproate, stiripentol, levetiracetam, and topiramate.

The median percent change from baseline in reduction of convulsive seizures in Dravet Syndrome for the CBD 20 mg/kg/day group was statistically superior to placebo (p=0.0123).

Following the trial statistical analysis was performed on the various patient groups to determine whether there was an interaction between any of the concomitant AEDs that the patients were taking. Data for the interaction between CBD and stiripentol (STP) are described below.

### Results

Table 6 below describes the percentage of patients that recorded a 50% reduction in convulsive seizures over the treatment period.

**Table 6: Interaction between CBD and other AEDs**

| **Combination of AEDs** | **Percentage of patients with greater than 50% reduction in seizures** |
|---|---|
| Stiripentol | 14% |
| Stiripentol + Clobazam | 8% |
| Stiripentol + CBD | 43% |
| Stiripentol + Clobazam + CBD | 26% |

As is shown, 14% of patients that were taking stiripentol and placebo experienced a greater than 50% reduction in the number of seizures from the number recorded during the 4 week baseline recording period.

Surprisingly, there was a reduction in efficacy in patients that were taking stiripentol and clobazam, where only 8% of these patients experienced a greater than 50% reduction in seizures from the baseline rate.

In the groups that were taking the test compound CBD, there was an increase in efficacy in both groups. It was found that 43% of patients that were on stiripentol and CBD obtained a greater than 50% reduction in convulsive seizures, whereas 26% of patients that were taking stiripentol, clobazam and CBD obtained a greater than 50% reduction in seizures.

### Conclusions

The increase in efficacy brought about by the addition of CBD to the AED stiripentol provides a useful combination of therapy. Such an increase in efficacy was unexpected.

Furthermore, those patients that are already taking a combination of stiripentol and clobazam may benefit from the inclusion of CBD as an adjunct therapy as such an inclusion has been found to reduce the number of seizures.

As can be seen in Table 3 both stiripentol and clobazam are commonly used AEDs in childhood epilepsy syndromes, furthermore they both work via enhancement of gamma-aminobutyric acid (GABA) A-receptor agonism.

Stiripentol is a positive allosteric modulator of GABA-A receptors in the brain that enhances the opening duration of the channel by binding to a site different than the benzodiazepine binding site. Reduced synaptosomal uptake of GABA and/or inhibition of GABA transaminase may also explain the role of stiripentol in reducing seizures.

Clobazam binds at distinct binding sites at the post-synaptic GABA receptor. These GABA receptors are in various locations in the CNS (limbic, reticular formation) and clobazam increases the duration of time for which the receptor is open. As a result, hyper polarization and stabilization of the membrane occur as the post-synaptic inhibitory effect of GABA is enhanced.

Combination of CBD with AEDs that work as GABA receptor agonists may therefore prove to be of particular benefit in the treatment of childhood epilepsy syndromes.

### EXAMPLE 2: DRUG-DRUG INTERACTION BETWEEN CANNABIDIOL (CBD) AND STIRIPENTOL (STP) IN HEALTHY VOLUNTEERS

As part of an open-label, fixed sequence, healthy volunteer trial the primary objective was to investigate the impact of CBD (750 mg twice daily) on the steady-state pharmacokinetics of stiripentol (STP) (750 mg) and the reciprocal effect on CBD, 7-hydroxy-cannabidiol (7-OH-CBD) and 7-carboxy-cannabidiol (7-COOH-CBD).

Analyte plasma concentrations were determined using validated bioanalytical methods. A secondary objective was to evaluate the safety and tolerability of CBD when co-administered with STP.

When CBD was combined with STP (12 subjects) there was a 1.28- to 1.55-fold increase in exposure (Cmax and AUCtau). Co-administration of STP with CBD had no effect on CBD exposure; however, STP reduced 7-OH-CBD and 7-COOH-CBD exposure by 29% and 13% respectively.

The most common adverse event (AE) was diarrhoea, none of the effects on analyte exposure observed were likely to be clinically relevant or correlated with incidence or severity of AEs.

### Conclusions

The above data demonstrate that the combination of CBD with STP provides a safe and efficacious combination treatment option.

## Claims

1. Cannabidiol (CBD) for use in the reduction of seizures in treatment-resistant Dravet syndrome, wherein the CBD is administered in combination with stiripentol.

2. Cannabidiol (CBD) for use according to claim 1, wherein the CBD is in the form of a highly purified extract of cannabis which comprises at least 98% (w/w) CBD.

3. Cannabidiol (CBD) for use according to claim 2, wherein the highly purified extract comprises less than 0.15% tetrahydrocannabinol (THC).

4. Cannabidiol (CBD) for use according to claim 2, wherein the extract further comprises up to 1% cannabidivarin (CBDV).

5. Cannabidiol (CBD) for use according to claim 1, wherein the CBD is present as a synthetic compound.

6. Cannabidiol (CBD) for use according to any of the preceding claims, wherein the dose of CBD is below 50 mg/kg/day.

7. Cannabidiol (CBD) for use according to any of the preceding claims, wherein the dose of CBD is greater than 10 mg/kg/day.

8. Cannabidiol (CBD) for use according to any of the preceding claims, wherein the dose of CBD is greater than 20 mg/kg/day.

## Patentansprüche

1. Cannabidiol (CBD) zur Verwendung bei der Reduzierung von Anfällen bei behandlungsresistentem Dravet-Syndrom, wobei das CBD in Kombination mit Stiripentol verabreicht wird.

2. Cannabidiol (CBD) zur Verwendung nach Anspruch 1, wobei das CBD in Form eines hoch aufgereinigten Cannabisextrakts ist, der mindestens 98 % (Gewicht/Gewicht) CBD umfasst.

3. Cannabidiol (CBD) zur Verwendung nach Anspruch 2, wobei der hoch aufgereinigte Extrakt weniger als 0,15 % Tetrahydrocannabinol (THC) umfasst.

4. Cannabidiol (CBD) zur Verwendung nach Anspruch 2, wobei der Extrakt ferner bis zu 1 % Cannabidivarin (CBDV) umfasst.

5. Cannabidiol (CBD) zur Verwendung nach Anspruch 1, wobei das CBD als synthetische Verbindung vorhanden ist.

6. Cannabidiol (CBD) zur Verwendung nach einem der vorherigen Ansprüche, wobei die CBD-Dosis unter 50 mg/kg/Tag ist.

7. Cannabidiol (CBD) zur Verwendung nach einem der vorherigen Ansprüche, wobei die CBD-Dosis mehr als 10 mg/kg/Tag ist.

8. Cannabidiol (CBD) zur Verwendung nach einem der vorherigen Ansprüche, wobei die CBD-Dosis mehr als 20 mg/kg/Tag ist.

## Revendications

1. Cannabidiol (CBD) destiné à être utilisé dans la réduction des convulsions dans le syndrome de Dravet résistant au traitement, ledit CBD étant administré en association avec le stiripentol.

2. Cannabidiol (CBD) destiné à être utilisé selon la revendication 1, ledit CBD se présentant sous la forme d'un extrait hautement purifié de cannabis qui comprend au moins 98 % (p/p) de CBD.

3. Cannabidiol (CBD) destiné à être utilisé selon la revendication 2, ledit extrait hautement purifié comprenant moins de 0,15 % de tétrahydrocannabinol (THC).

4. Cannabidiol (CBD) destiné à être utilisé selon la revendication 2, ledit extrait comprenant en outre jusqu'à 1 % de cannabidivarine (CBDV).

5. Cannabidiol (CBD) destiné à être utilisé selon la revendication 1, ledit CBD étant présent sous la forme d'un composé synthétique.

6. Cannabidiol (CBD) destiné à être utilisé selon l'une quelconque des revendications précédentes, la dose de CBD étant inférieure à 50 mg/kg/jour.

7. Cannabidiol (CBD) destiné à être utilisé selon l'une quelconque des revendications précédentes, ladite dose de CBD étant supérieure à 10 mg/kg/jour.

8. Cannabidiol (CBD) destiné à être utilisé selon l'une quelconque des revendications précédentes, ladite dose de CBD étant supérieure à 20 mg/kg/jour.
